(19) Europäisches Patentamt

European Patent Office

Office européen des brevets

(11) **EP 0 893 529 B1**

(12) **EUROPÄISCHE PATENTSCHRIFT**

(45) Veröffentlichungstag und Bekanntmachung des Hinweises auf die Patenterteilung:
**14.11.2001 Patentblatt 2001/46**

(51) Int Cl.[7]: **D04H 1/54**, A61F 13/15

(21) Anmeldenummer: **98112526.3**

(22) Anmeldetag: **07.07.1998**

(54) **Flüssigkeitsverteilvlies**

Liquid distribution mat

Nappe fibreuse facilitant la répartition d'un liquide

(84) Benannte Vertragsstaaten:
**BE DE ES FI FR GB IT SE**

(30) Priorität: **25.07.1997 DE 19732039**

(43) Veröffentlichungstag der Anmeldung:
**27.01.1999 Patentblatt 1999/04**

(73) Patentinhaber: **Christian Heinrich Sandler GmbH & Co. KG**
**95126 Schwarzenbach a d Saale (DE)**

(72) Erfinder:
• **Die Erfinder haben auf ihre Nennung verzichtet**

(74) Vertreter:
**LOUIS, PÖHLAU, LOHRENTZ & SEGETH**
**Postfach 3055**
**90014 Nürnberg (DE)**

(56) Entgegenhaltungen:
**EP-A- 0 539 703        WO-A-95/17542**
**WO-A-95/35081        WO-A-97/18783**
**US-A- 5 486 166**

**Beschreibung**

Die Erfindung betrifft Flüssigkeitsverteilvliese, wie sie insbesondere als Zwischenschicht zwischen Deckschicht und Flüssigkeits-Absorptionsschicht von Windeln oder wirkungsähnlichen, Flüssigkeit durch die Deckschicht aufnehmenden und in einer Absorptionsschicht speichernden Schichtanordnungen verwendet werden. Derartige Flüssigkeitsverteilvliese werden insbesondere in absorbierenden Hygieneprodukten eingesetzt, die im wesentlichen aus einer flüssigkeitsdurchlässigen, dem Körper zugewandten Deckschicht, einer flüssigkeitsundurchlässigen, der Wäsche zugewandten Oberflächenschicht und einem zwischen diesen beiden Schichten angeordneten Saugkörper bestehen, wobei das Flüssigkeitsverteilvlies dann zwischen Deckschicht und Saugkörper angeordnet ist.

Um für diese Zwecke eingesetzt werden zu können, müssen Flüssigkeitsverteilschichten bzw. -vliese gewisse Eigenschaften aufweisen. Wichtigste Eigenschaft ist eine gute Flüssigkeitsverteilung in Verbindung mit hoher Aufnahmegeschwindigkeit für die Flüssigkeit, da grosse anfallende Flüssigkeitsmengen ansonsten von der Absorptionsschicht nicht schnell genug aufgenommen werden könnten, was zu einer Leckage des Hygieneartikels führen kann. Weiterhin ist ein gutes Rücknässungsverhalten erforderlich, damit einmal von der Absorptionsschicht aufgenommene Flüssigkeit von dieser nicht wieder abgegeben wird und in Richtung Körper wandert. Das Rücknässungsverhalten wird unter anderem durch die Dicke und Druckstabilität des Flüssigkeitsverteilvlieses beeinflusst.

Die Absorptionsschichten bzw. Absorptionskörper moderner Hygieneprodukte enthalten augenblicklich neben einer Matrix aus hauptsächlich hydrophilen Zellulose-Pulpfasern einen grossen Anteil superabsorbierender, hydrogelbildender Partikel, so dass in den Saugkern eines Hygieneproduktes eintretende Flüssigkeit sehr intensiv gebunden werden kann. Ist bei einem Hygieneprodukt kein Flüssigkeitsverteilvlies vorhanden, so durchdringt die eintretende Flüssigkeit die Deckschicht überwiegend senkrecht, wodurch eine grosse Flüssigkeitsmenge auf eine relativ kleine Fläche verteilt wird und infolgedessen nur die genau unter dieser Fläche befindlichen superabsorbierenden Partikel zur Flüssigkeitsbindung in Aktion treten können..Die ausserhalb dieser Fläche befindlichen superabsorbierenden Partikel bleiben dagegen ungenutzt. Ein grosser Flüssigkeitsschwall auf einer kleinen Fläche bringt die von der Flüssigkeit benetzten superabsorbierenden Partikel sehr rasch zum Quellen, wodurch sich in der Aussenzone des Saugkerns des Hygieneproduktes eine Gelschicht bildet, welche das weitere Eindringen der Flüssigkeit in das Innere des Saugkerns verhindert. Diesen Zustand bezeichnet man als "Gel-Blocking". Er verhindert eine entsprechend gute Aufnahme spontan auftretender grosser Flüssigkeitsmengen, weshalb man bestrebt ist, eine spontan auftretende grosse Flüssigkeitsmenge vor dem Eindringen der Flüssigkeit in den Saugkern auf eine möglichst grosse Fläche zu verteilen, so dass die im Saugkern befindlichen, superabsorbierenden Partikel möglichst komplett ausgenutzt werden und die Flüssigkeitsmenge je Flächeneinheit möglichst gering wird, wodurch erreicht werden soll, dass sich das sogenannte "Gel-Blocking" nicht oder kaum negativ auswirkt.

Es ist weiter bekannt, dass im allgemeinen das Einbringen der Flüssigkeit in Hygieneprodukte in sehr kurzer Zeit und in grosser Menge erfolgt, weshalb es erforderlich ist, dass die relativ grosse Flüssigkeitsmenge nahezu augenblicklich vom Saugkörper aufgenommen und festgehalten wird. Es muss daher verhindert werden, dass sich unter der Deckschicht ein Flüssigkeitsstau bildet, da sich stauende Flüssigkeit nicht vom Saugkörper aufgenommen werden und aus dem Hygieneprodukt an unerwünschten Stellen herauslaufen würde. In diesem Zusammenhang ist es bereits bekannt (EP 0 539 703 A1), die Fasern einer Flüssigkeitsverteilschicht mit einer hydrophilen Avivage zu versehen. Diese Avivage kann jedoch ihre Aufgabe des Flüssigkeitstransportes nur so lange erfüllen, wie sie sich tatsächlich auf den Fasern befindet. Wenn mehrfach Flüssigkeit transportiert wird, besteht dabei die Gefahr, dass die Avivage abgewaschen wird und sich die Hygieneprodukte nach der zweiten, dritten oder gar vierten Flüssigkeitsbeaufschlagung nicht mehr trocken anfühlen, da die dann avivagefreie Polyolefinaussenschicht der Faser hydrophoben Charakter besitzt und die Flüssigkeit am Durchgang in Richtung Saugkörper hindert. Die Verfestigung der bekannten Flüssigkeitsverteilschicht erfolgt per Durchluft oder per Infrarot oder es wird ein spinngebundenes Vlies verwendet. Dies führt dazu, dass sich die eintretende Flüssigkeit gleichmässig in alle Richtungen ausbreitet, was häufig nicht sehr günstig ist. Ausserdem besitzt das bekannte Vlies nur geringe Druckstabilität, was zu verstärkter Rücknässung infolge Kompression führen kann.

Von Rücknässung spricht man bei einem Hygieneprodukt dann, wenn aus dem mit Flüssigkeit beladenen Saugkern unter Druckeinwirkung wieder Flüssigkeit in Richtung Körper abgegeben wird. Ziel moderner Hygieneprodukte ist ja, dass die Haut des Benutzers möglichst trocken bleibt, d.h. möglichst wenig Flüssigkeit in Richtung auf den Körper zurückgeführt wird. Es wird zwar ein Grossteil der Flüssigkeit im Saugkörper gut durch die superabsorbierenden Partikel festgehalten. Allerdings fühlt sich dabei der Saugkörper feucht an. Wenn nun der Saugkörper direkt mit der Deckschicht in Verbindung stünde, wäre die Gefahr gegeben, dass durch Kapillarkräfte Flüssigkeit aus dem Saugkörper in Richtung auf die Deckschicht zurückgesaugt werden würde, was zu einer Durchnässung der Deckschicht führen könnte. Aufgabe eines Flüssigkeitsverteilvlieses ist es somit auch, ein Zurücksaugen der Flüssigkeit aus dem Saugkörper in die Deckschicht zu verhindern.

Schliesslich sollen sämtliche modernen Faservliese sich natürlich reibungslos in der Hygieneindustrie verarbeiten lassen, wofür die mechanischen Eigenschaften der Vliesstoffe von grosser Bedeutung sind.

Aus vorstehenden Darlegungen ist ersichtlich, dass ein Flüssigkeitsverteilvlies einer Vielzahl von möglicherweise sogar widersprüchlichen Forderungen entsprechen muss. Es soll zum einen für eine gute Flüssigkeitsverteilung sorgen, weiter'eine kurze Durchdringungszeit für die Flüssigkeit gewährleisten, gute Rücknässungseigenschaften aufweisen, d.h. ein Rückströmen von Flüssigkeit aus dem Saugkörper in die Deckschicht verhindern, und schliesslich auch eine gute Stabilität in Verbindung mit ausgezeichneten mechanischen Eigenschaften besitzen.

Aus der WO 96/11107 ist bereits ein Vliesmaterial bekannt, welches die Aufgabe hat, die Flüssigkeitsverteilung zu verbessern. Dieses bekannte Vlies ist per Kalandertechnik verfestigt, wobei ein Prägekalander verwendet wird, der in sich geschlossene Prägebilder erzeugt, beispielsweise sechseckige Gebilde. In den vom Kalander geprägten Bereichen sind zusätzlich Durchbrechungen in dem bekannten Flüssigkeitsverteilvlies vorgesehen. Aufgrund der gesamten Konstruktion ist davon auszugehen, dass das bekannte Flüssigkeitsverteilvlies zwar die Ausbreitung von Flüssigkeit quer zur Vliesebene begünstigt. Eine Ausbreitung bzw. Verteilung von in das Vlies eindringener Flüssigkeit in der Hauptebene des Vlieses ist jedoch durch das Vorhandensein der in sich geschlossenen Prägeelemente, die zur Verfestigung des bekannten Vlieses dienen, ausgeschlossen. Infolgedessen sind die Einsatzgebiete des aus der WO 96/11107 bekannten Flüssigkeitsverteilvlieses sehr beschränkt.

Aufgabe der Erfindung ist es nun, ein Flüssigkeitsverteilvlies vorzuschlagen, welches den entsprechenden, vorstehend erläuterten Anforderungen an ein Flüssigkeitsverteilvlies in möglichst umfassender Weise entspricht.

Zur Lösung dieser Aufgabe wird erfindungsgemäss ein Flüssigkeitsverteilvlies vorgeschlagen, welches sich durch die Kombination folgender Eigenschaften auszeichnet:

a) Zusammensetzung aus gekräuselten thermoplastischen Fasern oder Fasermischungen mit einem überwiegenden Anteil an gekräuselten thermoplastischen Fasern,

b) Faserstärke zwischen 3 und 30 dtex, vorzugsweise zwischen 6,7 und 12 dtex,

c) hydrophile Avivierung der Fasern,

d) Flächengewicht zwischen 20 und 80 g/m$^2$,

e) Kompressibilität des Vlieses von weniger als 2,5 und

f) Kalanderverfestigung mittels eines beheizten Prägekalanders, dessen prägende Elemente (Prints) weniger als 25 %, vorzugsweise weniger als 15 % der Vliesoberfläche prägen, wobei das von den Prints gebildete Printmuster der Vliesoberfläche derart gestaltet ist, dass im wesentlichen in Maschinenrichtung verlaufende, durchgehende Kanäle aus nicht geprägtem Vliesmaterial gebildet sind.

Ein Faservlies mit den vorstehend genannten Eigenschaften weist infolge des gewählten Faserdurchmessers und der sich unter Berücksichtigung des Flächengewichtes ergebenden Vliesdichte eine für eine gute Flüssigkeitsverteilung günstige Porengrösse auf, wobei die Flüssigkeitsverteilung noch dadurch unterstützt werden kann, wenn - wie erfindungsgemäß bevorzugt - die Fasern der Fasermischung überwiegend in der der Fertigungs- und Verarbeitungsrichtung entsprechenden Längsrichtung (Maschinenrichtung) des Vlieses orientiert sind. Der Flüssigkeitstransport wird grundsätzlich durch die hydrophile Avivierung der Fasern begünstigt.

Die Verwendung vergleichsweise grober und drahtiger Fasern, die Art der Verfestigung des Vlieses sowie die sich ergebende nicht zu geringe Dicke des Flüssigkeitsverteilvlieses, die zweckmässig zwischen 0,3 und 3 mm, vorzugsweise zwischen 1 und 2,5 mm liegt, führen dazu, dass dieses eine gute Stabilität gegenüber Kompression besitzt, wodurch sich gute Rücknässungseigenschaften ergeben. Insgesamt darf, um ausreichende Rücknässungseigenschaften zu erzielen, die Kompressibilität des Vlieses nicht mehr als 2,5 betragen, d.h. das Vlies darf sich bei entsprechender Belastung nicht stärker als auf 40 % der Ausgangsdicke zusammendrücken lassen.

Aufgrund des entsprechenden Anteils von groben, hydrophilen Fasern, der Art der thermischen Kalanderverfestigung sowie des speziellen Gravurmusters des Flüssigkeitsverteilvlieses ergeben sich auch entsprechend gute mechanische Eigenschaften des Vliesstoffes, die dessen reibungslose Verarbeitung in der Hygieneindustrie sicherstellen. Dabei ist auch die hohe Lauflänge der Vliesstoffrollen günstig, die man erhält, wenn, wie erfindungsgemäss bevorzugt, das Flüssigkeitsverteilvlies eine Dicke von 0,3 bis 3 mm, vorzugsweise von 1,0 bis 2,5 mm, aufweist. Erstaunlicherweise genügen derart geringe Dicken bei dem Flüssigkeitsverteilvlies gemäss der Erfindung im Hinblick auf dessen ausgezeichnete Eigenschaften bezüglich Flüssigkeitsverteilung, Durchdringungszeit und Rücknässungseigenschaften.

Das spezielle Printmuster, welches bei der Kalanderverfestigung verwendet wird und in Maschinenrichtung verlaufende, durchgehende Kanäle aus nicht geprägtem Vliesmaterial bildet, bringt den Vorteil, dass das erfindungsgemässe Flüssigkeitsverteilvlies in der Lage ist, grosse anfallende Flüssigkeitsmengen in die Längsrichtung zu leiten.

Dies ist vor allem bei Verarbeitung des Vlieses in Hygieneartikeln von Bedeutung, da diese im allgemeinen in Schrittrichtung länger sind als senkrecht dazu. Würde bei solchen Artikeln die anfallende Flüssigkeitsmenge nicht bevorzugt in Schrittrichtung verteilt, wäre zu befürchten, dass die Hygieneartikel seitlich auslaufen. Selbstverständlich trägt zu der gerichteten Flüssigkeitsverteilung auch bei, dass die Fasern in die bevorzugte Verteilrichtung ausgerichtet sind, was durch Anwendung entsprechender Lege- oder Krempeltechniken erreicht werden kann, so dass der von den in Maschinenrichtung verlaufenden Kanälen, die durch die Kalander-Musterung erzeugt sind, bewirkte Effekt noch verstärkt wird.

Es ist zwar aus der WO 95/17542 bereits ein aus einem Vlies bestehendes, stoffähnliches Material bekannt, welches während der Veredelung des Ausgangsvliesstoffes mittels eines Prägekalanders verfestigt wird, wobei das Printmuster des Kalanders ebenfalls derart ausgebildet ist, dass sich in Maschinenrichtung verlaufende, nicht geprägte Kanäle ergeben. Diese spezielle Prägung erfolgt jedoch, damit der Vliesstoff durch Verstreckung entlang der Kalanderpunkte geöffnet werden kann, um so einen Rippeneffekt zu erhalten, der eine textile, strickstoffähnliche Optik hinsichtlich des fertigen, kalanderten und verstreckten Vliesstoffes ergibt. Irgendein Hinweis, dass diese spezielle Printmusterung günstig sein könnte, wenn es um die Ausbildung eines Flüssigkeitsverteilvlieses geht, ist der WO 95/17542 nicht zu entnehmen. Ziel des bekannten Vorgehens ist ausschliesslich die Erzeugung einer bestimmten, wertvoll erscheinenden Optik bei einem Vliesmaterial.

Aus der GB 2 288 412A ist ein Vlies bekannt, welches im wesentlichen aus thermoplastischen Fasern oder Fasermischungen mit einem überwiegenden Anteil an thermoplastischen Fasern besteht, wobei die Faserstärke weniger als 3,3 dtex beträgt, die Fasern mit einer hydrophilen Avivierung versehen sind und das Vlies ein Flächengewicht von 15,5 bis 35g/m$^2$ aufweist. Darüberhinaus ist das Vlies auch bereichsweise versiegelt, wobei die Versiegelungsrate zwischen 10 und 40% beträgt. Die entsprechenden Schweißpunkte sind bei dem bekannten Vlies, wie Figur 1 und 5 der GB 2 288 412A zeigen, im wesentlichen gleichmäßig über die Oberfläche des Vlieses verteilt, wobei entgegen dem Vlies gemäß der Erfindung keine die Flüssigkeit in bestimmte Richtung leitende Kanäle gebildet sind. Es ist weiterhin davon auszugehen, daß das Vlies gemäß GB 2 288 412A nicht die Eigenschaften eines Flüssigkeitsverteilvlieses hat. Es handelt sich bei dem bekannten Vlies vielmehr um ein Abdeckvlies, das den Zweck hat, Flüssigkeit beispielsweise von der Haut eines Benutzers einer entsprechenden Windel od. dgl. möglichst rasch abzuleiten und dem Speichervlies zuzuführen. Eine Flüssigkeitsverteilung im Sinne des Erfindungsvorschlages, nämlich parallel zur Vlies-Hauptebene, soll und kann mit dem bekannten Vlies nicht erreicht werden.

Aus der US 5 486 166 ist ein Flüssigkeits-Verteilvlies bekannt, welches ebenfalls aus thermoplastischen Fasern oder Fasermischungen mit einem hohen Anteil an thermoplastischen Fasern besteht, wobei die Faserstärke bei diesem bekannten Vlies mehr als 2 den, beispielsweise bis 16 den und vorzugsweise 3 bis 6 den betragen soll. Das bekannte Vlies ist auch mit einer hydrophilen Avivierung versehen und kann ein Flächengewicht von mehr als 20g/m$^2$ haben. In der US 5 486 166 ist jedoch weder auf die Bedeutung einer Begrenzung der Kompressibilität des Vlieses hingewiesen noch kann der Druckschrift ein Hinweis auf die Möglichkeit entnommen werden, das Vlies mittels eines beheizten Prägekalanders unter Ausbildung eines Printmusters bereichsweise zu versiegeln, wobei infolge des Printmusters im wesentlichen in Maschinenrichtung verlaufende, durchgehende Kanäle aus nichtgeprägtem Vliesmateril gebildet werden.

Das Flüssigkeitsverteilvlies gemäss der Erfindung kann aus den unterschiedlichsten thermoplastischen Fasern hergestellt werden. Besonders günstig ist es jedoch, wenn es aus folgenden Fasern oder einer Mischung hieraus besteht:

a) Polyolefinfasern einer Stärke von 3,3 bis 17 dtex, vorzugsweise von 6,7 bis 12 dtex,

b) Polyesterfasern (Hohl- oder Vollfasern) einer Stärke von 7 bis 17 dtex, vorzugsweise von 12 dtex,

c) Polyester/Copolyester-Bikomponentenfasern mit einer Stärke von 3,3 bis 17 dtex, wobei der Schmelzpunkt der niedriger schmelzenden Komponente um wenigstens 30 °C unter dem der höher schmelzenden Komponente liegt,

d) Polyester/Polyethylen-Bikomponentenfasern einer Stärke von 4,4 bis 17 dtex,

e) Polypropylen/Polyethylen-Bikomponentenfasern einer Stärke von 3,3 bis 17 dtex.

Die zweckmässigste Faser muss in Anpassung an das jeweils gewünschte Einsatzgebiet sowie die Bedingungen der Benutzung gewählt werden, wobei insbesondere auch spezielle Fasermischungen häufig sehr zweckmässig sein werden. Wichtig ist nur, dass der Anteil an thermoplastischen Fasern hoch genug ist, um im Rahmen der Kalanderverfestigung eine ausreichende Stabilität zu erreichen.

Es hat sich als günstig erwiesen, wenn die Fasern eine Länge von 30 bis 80 mm aufweisen. Derartige Fasern lassen sich bei der Herstellung gut verarbeiten und führen andererseits zu hervorragenden mechanischen Eigenschafen sowie auch einer guten Flüssigkeitsverteilung, da die einzelnen Fasern jeweils über eine vergleichsweise grosse Strecke im Vlies verlaufen.

Als Bikomponentenfasern werden vorteilhafterweise solche des Mantel/Kern-Typs verwendet, weil deren Einsatz zu einer besonders guten Bindung des Vlieses und damit entsprechend mechanischer Festigkeit führt.

Günstig ist es in diesem Zusammenhang, wenn die im Gebrauch einer Absorptionsschicht (Saugschicht) zugekehrte Oberfläche des Flüssigkeitsverteilvlieses mittels der beheizten Kalanderwalze oder durch eine Siebtrommel zusätzlich geglättet ist. Die durch die Glättung entstehende Verdichtung der zum Saugkörper weisenden Oberfläche des Flüssigkeitsverteilvlieses begünstigt ebenfalls die Flüssigkeitsverteileigenschaften.

Das Printmuster kann, abhängig von den jeweiligen Anforderungen an das Flüssigkeitsverteilvlies, unterschiedlich gestaltet sein.

Zweckmässig wird ein Printmuster verwendet, das mittels eines Prägekalanders erzeugt ist, der eine gemusterte Walze einer Gravurtiefe von wenigstens 1 mm, vorzugsweise von etwa 1,2 mm aufweist. Diese Gravurtiefe liegt etwas höher als die üblicherweise bei der Vliesverfestigung verwendeten Gravurhöhen von 0,6 bis 0,8 mm. Hierdurch wird erreicht, dass bei Verarbeitung von Vliesen mit relativ hohem Flächengewicht keine zu grosse Masse an Fasern in die Gravurzwischenräume gepresst wird, wodurch sich eine hinreichende jedoch nicht zu grosse Enddicke des Vliesstoffes bei einem Arbeiten gemäss der Erfindung ergibt.

Die Gravurtiefe bestimmt in Verbindung mit der Pressfläche des Kalanders, die erfindungsgemäss bei weniger als 25 %, vorzugsweise weniger als 15 % liegt, die Dicke des erzeugten Vlieses, wobei eine hohe Pressfläche des Kalanders ein dünneres Vlies erzeugt, eine geringere Pressfläche dagegen ein etwas dickeres Vlies, da die Fasern an den unverfestigten, durch die Gravurzwischenräume gebildeten Stellen noch einen gewissen Bausch besitzen, während sie an den durch die Pressfläche des Kalanders verschweissten Stellen folienartig verschmolzen sind. In gleiche Weise erfolgt bei Verwendung der erfindungsgemässen Gravurtiefe keine allzu starke Verdichtung der in die Gravurzwischenräume gepressten Fasermasse.

Als für die Zwecke der Erfindung besonders geeignet haben sich Printmuster aus 1 bis 80, vorzugsweise 2 bis 15 Prints pro cm$^2$ erwiesen.

Es ist weiter nach der Erfindung vorgesehen, dass die Prints von Stäbchen oder von langgestreckten Rauten oder Ellipsen gebildet sind, deren Länge wenigstens der 1,5-fachen Breite entspricht. Derartige Printmuster wirken deutlich im Sinne einer bevorzugten Flüssigkeitsverteilung in Richtung der Längsachse der Stäbchen, Rauten oder Ellipsen, so dass auf diese Weise die Flüssigkeitsverteileigenschaften des Vlieses positiv beeinflusst werden können.

Für die Anordnung derartiger Stäbchen, Rauten oder Ellipsen gibt es unterschiedliche, verschieden günstige Möglichkeiten, die wiederum von dem jeweiligen Einsatzgebiet abhängen. Vorzugsweise ist jedoch so vorzugehen, dass die Längsachse der Prints innerhalb eines Winkels von ± 75°, vorzugsweise von ± 65° gegenüber der Maschinenrichtung des Vlieses verläuft, wobei es für bestimmte Einsatzfälle besonders günstig sein kann, wenn die Längsachse der Prints etwa in Maschinenrichtung des Vlieses zeigt.

Schliesslich ist nach der Erfindung auch noch vorgesehen, dass die Prints von Quadraten gebildet sind, die derart ausgerichtet sind, dass eine Diagonale etwa in Maschinenrichtung des Vlieses verläuft. Wichtig ist dabei jedoch, dass die die Prints bildenden Quadrate so angeordnet sind, dass sich die längs in Maschinenrichtung des Grundvlieses vorgesehenen, zur Flüssigkeitsverteilung dienenden Kanäle ergeben.

Nachstehend wird ein Ausführungsbeispiel eines Flüssigkeitsverteilvlieses gemäss der Erfindung unter Bezugnahme auf die Zeichnung näher erläutert.

Es zeigen -:

Figuren 1 bis 3       erläuternde Skizzen zum sog. "Kanga"-Test zur Ermittlung der Durchdringungszeit,

Figur 4       um etwa 10 % verkleinert ein Printmuster, wie es bei dem nachstehend erläuterten Ausführungsbeispiel zur Kalanderverfestigung verwendet wurde,

Figur 5       schematisch drei mögliche Ausführungsformen von Printmustern und

Figur 6       eine Schemaskizze zur Erläuterung der zweckmässigen Ausrichtung der Prints.

Es wurde gemäss folgendem
    Ausführungsbeispiel
ein Flüssigkeitsverteilvlies hergestellt -:

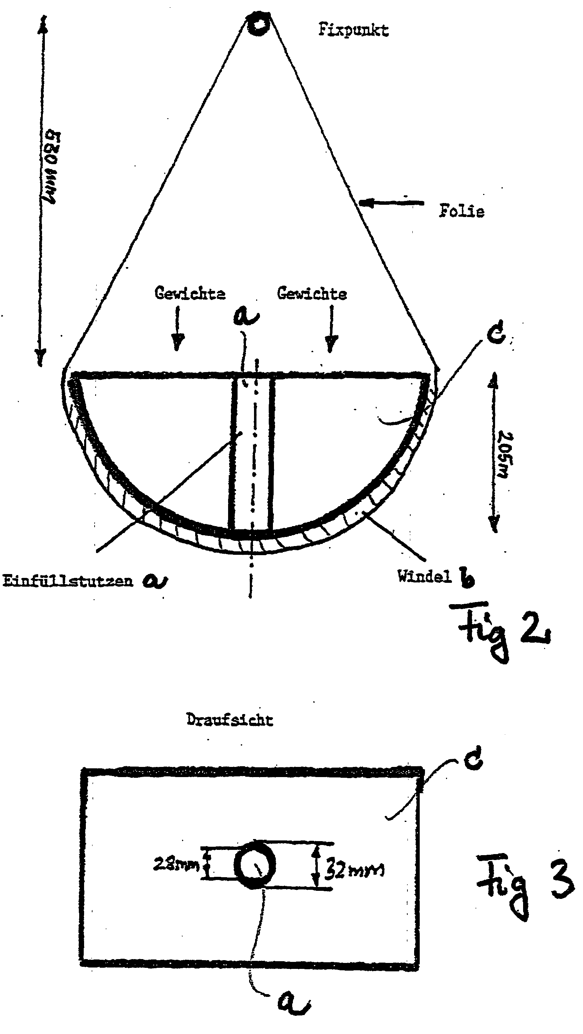

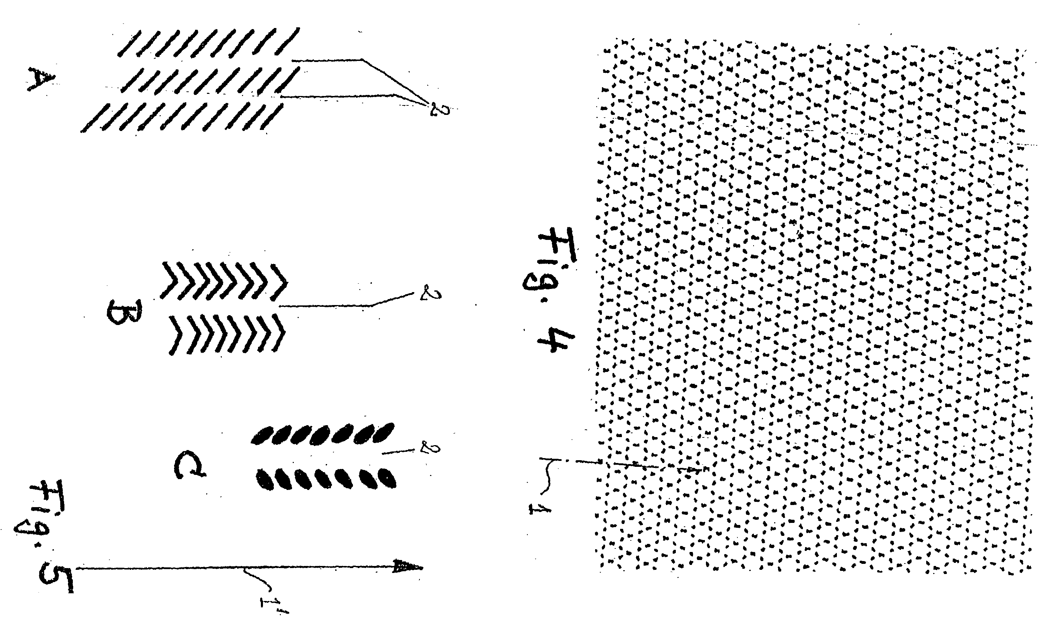

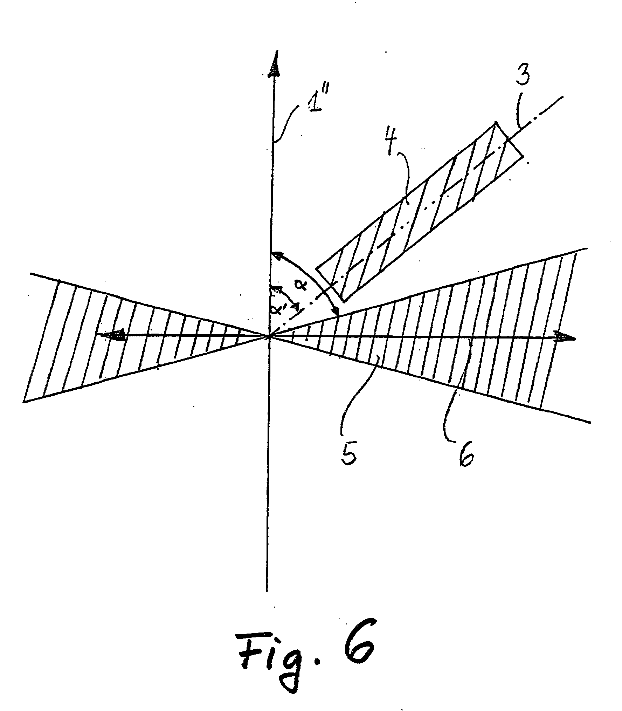

Eine Fasermischung aus 50 % Polyester/Copolyester-Bikomponentenfasern des Mantel/Kern-Typs einer Stärke von 5,3 dtex wurden mit 50 % Polyester-Vollfasern einer Stärke von 12 dtex gemischt und zu einem Faserflor mit einem Gewicht von 45 g/m$^2$ gekrempelt. Die Fasern waren weitgehend in Maschinenrichtung orientiert. Anschliessend wurde der Faserflor per Kalander bei 138 °C auf Glattwalze und Gravurwalze mit einem Liniendruck von 70 daN mit dem in Figur 4 gezeigten Muster verfestigt. Die Gravur der Gravurwalze war derart, dass der Pressflächenanteil 6,9 % und die Gravurtiefe 1,2 mm betrugen. Die schrägstehenden Gravurelemente (Prints) verliefen unter etwa $\pm$ 45° bzw. $\pm$ 225°zur Maschinenrichtung.

Der so verfestigte Vliesstoff wurde über Kühlwalzen gekühlt und gewickelt und kann so einer Konfektionsmaschine für Hygieneartikel vorgelegt werden.

Zur Untersuchung seiner Flüssigkeitsverteileigenschaften wurde der so erhaltene Vliesstoff als Flüssigkeitsverteilvlies zwischen die Deckschicht und die Absorptionsschicht bzw. den Saugkörper einer Babywindel eingebaut und es wurden dann die Eigenschaften des Vliesstoffes wie folgt bestimmt:

| Dicke | 1,35 mm |
|---|---|
| Kompressibilität | 1,72 |
| Durchdringungszeit | |
|     erste Flüssigkeitsbeaufschlagung | 17 sec. |
|     zweite Flüssigkeitsbeaufschlagung | 45 sec. |
|     dritte Flüssigkeitsbeaufschlagung | 75 sec. |

In diesem Zusammenhang stellt die Kompressibilität ein Mass für gute Rücknässungseigenschaften dar. Die Durchdringungszeit 'ist ein Mass für die Flüssigkeitsverteilung, da die Durchdringungszeit umso kürzer ist, je rascher die auf das Hygieneprodukt im Bereich der Deckschicht aufgebrachte Flüssigkeit von der Absorptionsschicht aufgenommen wird. Die Aufnahme erfolgt jedoch umso rascher, je besser die Flüssigkeit über eine möglichst grosse Fläche verteilt wird.

Zur Verdeutlichung der Wirksamkeit des Flüssigkeitsverteilvlieses gemäss der Erfindung wurde die Durchdringungszeit bei einer ansonsten der Babywindel des Ausführungsbeispiels entsprechenden Babywindel, jedoch ohne Flüssigkeitsverteilschicht gemessen.

Dabei ergaben sich für die Durchdringungszeit folgende Werte:

| erste Flüssigkeitsbeaufschlagung | 38 sec. |
|---|---|
| zweite Flüssigkeitsbeaufschlagung | 165 sec. |
| dritte Flüssigkeitsbeaufschlagung | 287 sec. |

Hieraus ist ersichtlich, dass das Flüssigkeitsverteilvlies gemäss der Erfindung dazu führt, dass die Flüssigkeit wesentlich rascher verteilt bzw. von der Absorptionsschicht aufgenommen wird.

Zur Messung der Eigenschaften wurden folgende Mess- bzw. Prüfmethoden verwendet:

**Dicke:**

Messung nach DIN 53855 T1

**Kompressibilität:**

Hierin soll gemäss der Erfindung das Verhältnis der Dicke gemessen nach DIN 53855 T2 zur Dicke gemessen nach DIN 53855 T1 verstanden werden, also

$$Kompressibilität =$$

$$Dicke\ (DIN\ 53855\ T2)\ /\ Dicke\ (DIN\ 53855\ T1)$$

Die Dicke nach DIN 53855 T2 wird mit einer Vorlast von 0,2 cN/cm$^2$ gemessen, die Dicke nach DIN 53855 T1 mit einer Vorlast von 5 cN/cm$^2$. Das Vlies wird bei höherer Vorlast (DIN 53855 T1) stärker zusammengedrückt und weist infolgedessen eine geringere gemessene Dicke auf. Das Verhältnis der Dicke nach DIN 53855 T2 zur Dicke nach DIN 53855 T1 ergibt somit ein Mass für die Stabilität des Vliesstoffes bei Krafteinwirkung auf die Fläche.

**Durchdringungszeit:**

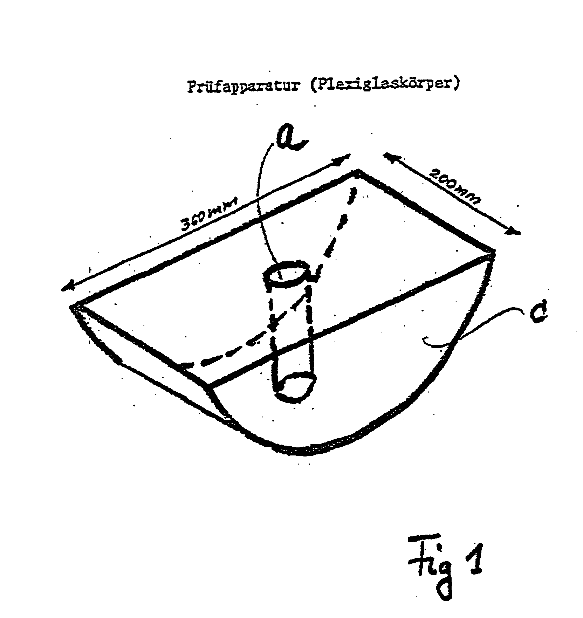

Hier wurde der sogenannte "Kanga"-Test der Firma Chemische Fabrik Stockhausen, Krefeld, verwendet, der nachstehend anhand der Figuren 1 bis 3 erläutert werden soll.

Die Kanga-Testmethode dient unter anderem zur Beurteilung der Durchdringungszeit eines Hygieneproduktes, im Ausführungsbeispiel einer Babywindel.

Es wird ein Testkörper (Windel) wie folgt vorbereitet.

Das auf der Windel b befindliche Abdeckvlies (Deckschicht) wird vorsichtig von dem Saugkörper abgelöst, wobei bei Messung der vorstehend erwähnten Durchdringungszeit eine handelsübliche Windel der Firma Carter Holt, Harvey, NZ, Marke "Treasures", Type "Trim Care", Größe: "X-large" verwendet wurde. Nun wird das zu prüfende Flüssigkeitsverteilvlies in entsprechender Grösse auf den Saugkörper aufgelegt. Über das Flüssigkeitsverteilvlies wird nun ein Referenz-Abdeckvlies (hier Typ Sawabond [eingetragene Marke der Anmelderin] 4302 mit 18 g/m$^2$) gelegt und mit einem Klebeband am Rand auf der gesamten Windel fixiert.

Der so vorbereitete Testkörper (Windel) b wird nun in den sogenannten "Kanga"-Tester eingelegt, wobei Einzelheiten des Testkörpers c aus der beiliegenden Skizze gemäss Figur 1 bis 3 ersichtlich sind.

Entsprechend der der jeweiligen Windelgrösse zugeordneten Gewichtsklasse (siehe nachstehende Tabelle) erfolgt die Gewichtsbelastung der Testapparatur:

| | |
|---|---|
| mini Windel | 4,5 kg |
| midi Windel | 6,5 kg |
| maxi Windel | 12,5 kg |
| X-large Windel | 15,0 kg |
| Junior Windel | 18,5 kg |

Bei den Versuchen wurde jeweils, wie oben erwähnt, eine "X-large"-Windel und entsprechend eine Gewichtsbelastung von 15 kg verwendet.

Nach Einbringung der Testwindel b in die Apparatur c und Gewichtsbelastung erfolgt nun eine definierte Flüssigkeitsbeaufschlagung mit Urin-Ersatzflüssigkeit (0,9 %ige NaCl-Lösung), wobei auch die Flüssigkeitsmenge entsprechend der Windelgrösse zu wählen ist.

| | |
|---|---|
| mini Windel | 40 g |
| midi Windel | 60 g |
| maxi Windel | 80 g |
| X-large Windel | 100 g |
| Junior Windel | 120 g |

Entsprechend der gewählten "X-large"-Windel wurde für die Versuchsreihe eine Flüssigkeitsmenge von 100 g verwendet.

Die aufzubringende Flüssigkeitsmenge wird mittels eines Messzylinders durch den Einfüllstutzen a der Testapparatur c zugegeben. Mittels einer Stoppuhr wird die Einsickerzeit der Flüssigkeit vom Zugabebeginn bis zum vollständigen Einsickern der Testflüssigkeit in den Saugkörper gemessen. Diese Zeit entspricht der ersten Durchdringungszeit.

Nach einer Wartezeit von 30 Minuten erfolgt eine zweite Zugabe der gleichen Flüssigkeitsmenge. Die Zeit vom Beginn der Zugabe dieser zweiten Flüssigkeitsmenge bis zu deren vollständigem Einsickern entspricht der zweiten Durchdringungszeit.

Nach einer Wartezeit von weiteren 30 Minuten erfolgt nun letztlich die dritte Zugabe der entsprechenden Flüssigkeitsmenge,. wobei die Zeit ab Beginn der Zugabe der dritten Flüssigkeitsmenge bis zu deren vollständigem Einsickern der dritten Durchdringungszeit entspricht.

Da die Testapparatur c aus einem durchsichtigen Material, z.B. Plexiglas, besteht, kann genau beobachtet werden, wie lange es dauert, bis die Urin-Ersatzflüssigkeit vollständig aus dem Einfüllstutzen a verschwunden, d.h. von der Windel b bzw. deren Saugkörper aufgenommen worden ist.

Printmuster:

Das in Figur 4 gezeigte Printmuster besteht aus drei Gruppen von Prints, nämlich Prints, die im wesentlichen in Maschinenrichtung (Pfeil 1) verlaufen, sowie Prints, die gegenüber der Maschinenrichtung 1 in einem Winkel von etwa ± 45° bzw. ± 225°angeordnet sind. Länge und Plazierung der Prints sind allerdings so gewählt, dass sich (wie durch den Pfeil 1 in Figur 4 angedeutet) im wesentlichen in Maschinenrichtung verlaufende Kanäle ergeben. In Verbindung mit der Ausrichtung der Fasern des Vlieses in der Hauptsache in Maschinenrichtung erhält man auf diese Weise ein Flüssigkeitsverteilvlies, bei dem die Flüssigkeitsverteilung in Maschinenrichtung wesentlich stärker ausgeprägt ist als quer zu der Maschinenrichtung 1.

In Figur 5 sind drei Beispiele für ein Printmuster gezeigt, wie es anstelle des Musters der Figur 4 für ein Flüssigkeitsverteilvlies gemäss der Erfindung eingesetzt werden könnte.

Das Printmuster A in Figur 5 besteht im wesentlichen aus zueinander parallelen Stäben, die gegenüber der durch den Pfeil 1' in Figur 5 angedeuteten Maschinen- bzw. Laufrichtung unter einem Winkel von etwa 60° angeordnet sind. Die Stäbe des Printmusters A in Figur 5 sind in Gruppen in Laufrichtung 1' hintereinander angeordnet, jedoch gleichzeitig derart plaziert, dass zwischen den einzelnen Gruppen der das Printmuster bildenden Stäbe in Laufrichtung 1' verlaufende Kanäle 2 gebildet sind, die zum raschen Flüssigkeitstransport in Laufrichtung 1' dienen. Das Printmuster B in Figur 5 besteht jeweils aus winkelförmigen Elementen, wobei auch hier die Anordnung derart ist, dass im wesentlichen übereinstimmende winkelförmige Elemente in Laufrichtung 1' gruppenweise aufeinanderfolgend angeordnet sind. Gleichzeitig wird jedoch zwischen den einzelnen Gruppen ein Abstand eingehalten, so dass ebenfalls in Laufrichtung 1' verlaufende Kanäle 2 zum Flüssigkeitstransport gebildet werden.

Die einzelnen Prints des Printmusters C in Figur 5 sind von länglich ovalen Elementen gebildet, wobei - wie die Zeichnung erkennen lässt - die Längsachse der einzelnen Elemente des Printmusters C deutlich grösser ist als deren Querachse. Erfindungsgemäss sollte die Länge der längsovalen bzw. elliptischen Prints des Printmusters C wenigstens das 1,5 fache ihrer Breite betragen.

Auch bei dem Printmuster C ist die Anordnung der Prints wiederum in Gruppen in Längsrichtung 1' vorgesehen, wobei zwischen den Gruppen ein hinreichender Abstand zur Bildung von Längskanälen 2 gewährleistet ist.

Ausserdem ist die Längsachse der Prints des Printmusters C wiederum unter einem Winkel von etwa 45° bis 75° gegenüber der Maschinenrichtung 1' verlaufend angeordnet.

In Figur 6 wird schliesslich veranschaulicht, wie die Längsachse 3 der Prints 4 gegenüber der Maschinenrichtung 1" zweckmässig ausgerichtet sein sollte.

Die besten Flüssigkeitstransportverhältnisse in Maschinenrichtung 1" erhält man nämlich, wenn die Längsachse 3 der einzelnen Prints 4 (in Figur 6 eines stäbchenförmigen Prints) unter einem Winkel $\alpha$ von etwa 0° bis 75° gegenüber der Maschinenrichtung 1" angeordnet ist.

Bei dem in Figur 6 schematisch gezeigten Ausführungsbeispiel beträgt der Winkel a' etwa 53°.

Der schraffiert gekennzeichnete Winkelbereich 5 (etwa 75° bis 105° gegenüber der Maschinenrichtung 1") ist für die Zwecke der Erfindung ungünstig, weil bei einer Anordnung der Längsachse der Prints 4 in diesem Winkelbereich eine Verteilung der Flüssigkeit in der durch den Doppelpfeil 6 gekennzeichneten Querrichtung des Vlieses zu stark begünstigt würde.

Es ist selbstverständlich denkbar, die Printmuster abweichend von den skizzieren Ausführungsbeispielen in Anpassung an die jeweiligen Erfordernisse zu variieren, wobei sowohl die gegenseitige Anordnung der Prints als auch deren Ausrichtung und Gestaltung sowie schliesslich die Printdichte verändert werden können.

Beispielsweise könnte das Printmuster derart ausgeführt werden, dass die Prints von Quadraten gebildet sind. In einem derartigen Fall müssten die Quadrate dann derart ausgerichtet sein, dass eine ihrer Diagonalen etwa in Maschinenrichtung des Vlieses verläuft, wobei durch entsprechende Grösse und Ausrichtung sowie Anordnung der quadratischen Prints dafür gesorgt werden müsste, dass sich wiederum in Maschinenrichtung verlaufende Längskanäle ergeben.

Zweckmässig wird man allerdings das Printmuster so gestalten, dass auf einem cm$^2$ 1 bis 80, vorzugsweise 2 bis 15 Prints vorhanden sind, wobei die insgesamt bei der Kalanderverfestigung von den Prints beaufschlagte Pressfläche weniger als 25 %, vorzugsweise weniger als 15 % der gesamten Vliesoberfläche beträgt. Wenn man diese Bedingungen einhält, lässt sich ohne weiteres ein Muster erzeugen, das einerseits ausreichend grosse Kanäle zum Flüssigkeitstransport ergibt, andererseits auch genügend nicht verfestigtes Vlies belässt, um hinreichende Saugfähigkeit und genügende Dicke sowie passende Kompressibilität zu erzeugen. Gleichzeitig erhält das Flüssigkeitsverteilvlies die für die Verarbeitung, beispielsweise in einem Hygieneprodukt, erforderliche Stabilität.

**Patentansprüche**

1.   Flüssigkeitsverteilvlies, insbesondere zur Verwendung als Zwischenschicht zwischen Deckschicht und Flüssigkeits- Absorptionsschicht von Windeln oder wirkungsähnlichen, Flüssigkeit durch die Deckschicht aufnehmenden und in der Absorptionsschicht speichernden Schichtanordnungen,
**gekennzeichnet durch**
die Kombination folgender Eigenschaften:

a) Zusammensetzung aus gekräuselten, thermoplastischen Fasern oder Fasermischungen mit einem überwiegenden Anteil an gekräuselten thermoplastischen Fasern,

b) Faserstärke zwischen 3 und 30 dtex, vorzugsweise zwischen 6,7 und 12 dtex,

c) hydrophile Avivierung der Fasern,

d) Flächengewicht zwischen 20 und 80 g/m$^2$,

e) Kompressibilität des Vlieses von weniger als 2,5 und

f) Kalanderverfestigung mittels eines beheizten Prägekalanders, dessen prägende Elemente (Prints 4) weniger als 25 %, vorzugsweise weniger als 15 %, der Vliesoberfläche prägen, wobei das von den Prints (4) gebildete Printmuster (A, B, C; Figur 4) der Vliesoberfläche derart gestaltet ist, dass im wesentlichen in Maschinenrichtung (1, 1', 1") verlaufende, durchgehende Kanäle (2) aus nicht geprägtem Vliesmaterial gebildet sind.

2. Flüssigkeitsverteilvlies nach Anspruch 1,
   **dadurch gekennzeichnet,**
   **dass** es aus folgenden Fasern oder einer Mischung hieraus besteht:

   a) Polyolefinfasern einer Stärke von 3,3 bis 17 dtex, vorzugsweise von 6,7 bis 12 dtex,

   b) Polyesterfasern einer Stärke von 7 bis 17 dtex, vorzugsweise von 12 dtex,

   c) Polyester/Copolyester-Bikomponentenfasern mit einer Stärke von 3,3 bis 17 dtex, wobei der Schmelzpunkt der niedriger schmelzenden Komponente um wenigstens 30 °C unter dem der höher schmelzenden Komponente liegt,

   d) Polyester/Polyethylen-Bikomponentenfasern einer Stärke von 4,4 bis 17 dtex,

   e) Polypropylen/Polyethylen-Bikomponentenfasern einer Stärke von 3,3 bis 17 dtex.

3. Flüssigkeitsvlies nach Anspruch 1 oder 2,
   **dadurch gekennzeichnet,**
   **daß** die Fasern überwiegend in der der Fertigungs- und Verarbeitungsrichtung entsprechenden Längsrichtung (Maschinenrichtung 1, 1', 1") des Vlieses orientiert sind.

4. Flüssigkeitsverteilvlies nach einem der Ansprüche 1 bis 3,
   **dadurch gekennzeichnet,**
   **dass** die Fasern eine Länge von 30 bis 80 mm aufweisen.

5. Flüssigkeitsverteilvlies nach einem der vorhergehenden Ansprüche,
   **dadurch gekennzeichnet,**
   **dass** die Bikomponentenfasern solche des Mantel/KernTyps sind.

6. Flüssigkeitsverteilvlies nach einem der vorhergehenden Ansprüche,
   **dadurch gekennzeichnet,**
   **dass** die im Gebrauch einer Absorptionsschicht zugekehrte Oberfläche des Flüssigkeitsverteilvlieses mittels der beheizten Kalanderwalze oder einer Siebtrommel geglättet ist.

7. Flüssigkeitsverteilvlies nach einem der vorhergehenden Ansprüche,
   **gekennzeichnet durch**
   eine Dicke von 0,3 bis 3 mm, vorzugsweise von 1 bis 2,5 mm.

8. Flüssigkeitsverteilvlies nach einem der vorhergehenden Ansrüche,
   **gekennzeichnet durch**
   ein Printmuster (A, B, C; Figur 4), das mittels eines Prägekalanders erzeugt ist, der eine gemusterte Walze einer Gravurtiefe von wenigstens 1 mm, vorzugsweise etwa 1,2 mm aufweist.

9. Flüssigkeitsverteilvlies nach einem der vorhergehenden Ansprüche,

**dadurch gekennzeichnet,**
**dass** das Printmuster (A, B, C; Figur 4) aus 1 bis 80, vorzugsweise 2 bis 15 Prints (4) pro cm$^2$ besteht.

10. Flüssigkeitsverteilvlies nach einem der vorhergehenden Ansprüche,
**dadurch gekennzeichnet,**
**dass** die Prints (4) von Stäbchen oder von langgestreckten Rauten oder Ellipsen gebildet sind, deren Länge wenigstens der 1,5-fachen Breite entspricht.

11. Flüssigkeitsverteilvlies Anspruch 10,
**dadurch gekennzeichnet,**
**dass** die Längsachse (3) der Prints (4) innerhalb eines Winkels von $\pm$ 75°, vorzugsweise von $\pm$ 65° gegenüber der Maschinenrichtung (1, 1', 1") des Vlieses verläuft.

12. Flüssigkeitsverteilvlies nach Anspruch 10,
**dadurch gekennzeichnet,**
**dass** die Längsachse der Prints (4) etwa in Maschinenrichtung (1, 1', 1") des Vlieses zeigt.

13. Flüssigkeitsverteilvlies nach einem der Ansprüche 1 bis 9,
**dadurch gekennzeichnet,**
**dass** die Prints (4) von Quadraten gebildet sind, die derart ausgerichtet sind, dass eine Diagonale etwa in Maschinenrichtung (1, 1', 1") des Vlieses verläuft.

**Claims**

1. Liquid distribution web, especially for use as an interlayer between the top layer and the liquid absorption layer of diapers or similar layered arrangements that acquire liquid through the top layer and store it in the absorption layer, **characterized by** the combination of the following properties:

   a) composition comprising crimped thermoplastic fibres or fibre blends having a predominant fraction of crimped thermoplastic fibres,
   b) fibre linear density between 3 and 30 dtex, preferably between 6.7 and 12 dtex,
   c) hydrophilic finish on the fibres,
   d) basis weight between 20 and 80 g/m$^2$,
   e) web compressibility less than 2.5 and
   f) calender consolidation using a heated embossing calender whose embossing elements (prints 4) emboss less than 25%, preferably less than 15%, of the web surface, the prints (4) forming a print pattern (A, B, C; Figure 4) on the web surface such that through channels (2) extending essentially in the machine direction (1, 1', 1") are formed of non-embossed web material.

2. Liquid distribution web according to Claim 1, **characterized in that** it consists of the following fibres or a blend thereof:

   a) polyolefin fibres having a linear density of 3.3 to 17 dtex, preferably of 6.7 to 12 dtex,
   b) polyester fibres having a linear density of 7 to 17 dtex, preferably of 12 dtex,
   c) polyester-copolyester bicomponent fibres having a linear density of 3.3 to 17 dtex, the melting point of the lower-melting component being at least 30°C below that of the higher-melting component,
   d) polyester-polyethylene bicomponent fibres having a linear density of 4.4 to 17 dtex,
   e) polypropylene-polyethylene bicomponent fibres having a linear density of 3.3 to 17 dtex.

3. Liquid distribution web according to Claim 1 or 2, **characterized in that** the fibres are predominantly oriented in the web's longitudinal direction (machine direction 1, 1', 1") which corresponds to the production and processing direction.

4. Liquid distribution web according to any of Claims 1 to 3, **characterized in that** the fibres are 30 to 80 mm in length.

5. Liquid distribution web according to any preceding claim, **characterized in that** the bicomponent fibres are of the sheath-core type.

**6.** Liquid distribution web according to any preceding claim, **characterized in that** that surface of the liquid distribution web which in use faces an absorption layer has been smoothed using the heated calender roll or a sieve drum.

**7.** Liquid distribution web according to any preceding claim, **characterized by** a thickness of 0.3 to 3 mm, preferably of 1 to 2.5 mm.

**8.** Liquid distribution web according to any preceding claim, **characterized by** a print pattern (A, B, C; Figure 4) which is generated using an embossing calender comprising a patterned roll having an engraved depth of at least 1 mm, preferably about 1.2 mm.

**9.** Liquid distribution web according to any preceding claim, **characterized in that** the print pattern (A, B, C; Figure 4) consists of 1 to 80, preferably 2 to 15, prints (4) per $cm^2$.

**10.** Liquid distribution web according to any preceding claim, **characterized in that** the prints (4) are formed by rods or by elongated diamonds or ellipses whose length is at least 1.5 times their width.

**11.** Liquid distribution web according to Claim 10, **characterized in that** the longitudinal axis (3) of the prints (4) extends within an angle of $\pm 75°$, preferably of $\pm 65°$, with regard to the machine direction (1, 1', 1") of the web.

**12.** Liquid distribution web according to Claim 10, **characterized in that** the longitudinal axis of the prints (4) points approximately in the machine direction (1, 1', 1") of the web.

**13.** Liquid distribution web according to any of Claims 1 to 9, **characterized in that** the prints (4) are formed by squares which are aligned in such a way that one diagonal extends approximately in the machine direction (1, 1', 1") of the web.

**Revendications**

**1.** Nappe fibreuse pour la répartition d'un liquide, destinée en particulier à son application comme couche intermédiaire entre une couche de couverture et une couche d'absorption de liquide de couches ou de structures en couches à actions semblables recueillant un liquide à travers la couche de couverture et l'accumulant dans la couche d'absorption, **caractérisée par** la combinaison suivante de propriétés :

a) constitution, à partir de fibres thermoplastiques ondulées ou de mélanges de fibres comportant une proportion prépondérante de fibres thermoplastiques ondulées;
b) résistance des fibres comprise entre 3 et 30 dtex, de préférence entre 6,7 et 12 dtex;
c) avivage hydrophile des fibres ;
d) densité superficielle entre 20 et 80 g/$m^2$ ;
e) compressibilité de la nappe de fibres inférieure à 2,5 ; et
f) consolidation par calandrage au moyen d'une calandre d'impression chauffée, dont l'élément imprimant (4 impressions) est inférieur à 25%, de préférence inférieur à 15%, de la superficie de la nappe, les motifs d'impression créés par les impressions (4) (A, B, C ; figure 4) de la surface de la nappe de fibres étant agencés de manière telle que des canaux continus (2) s'étendant dans la direction de la machine (1, 1', 1") soient formés par de la matière de la nappe non imprimée.

**2.** Nappe fibreuse pour la répartition d'un liquide selon la revendication 1, **caractérisée en ce qu'**elle se compose des fibres ou d'un mélange de fibres suivant :

a) fibres de polyoléfine d'une résistance de 3,3 à 17 dtex, de préférence de 6,7 à 12 dtex ;
b) fibres de polyester d'une résistance de 7 à 17 dtex, de préférence de 12 dtex ;
c) fibres à bi-composants polyesters / co-polyesters d'une résistance de 3,3 à 17 dtex, dans lesquels le point de fusion du composant fondant le plus bas est inférieur d'au moins 30°C à celui du composant fondant le plus haut ;
d) fibres à bi-composants de polyesters / polyéthylène d'une résistance de 4,4 à 17 dtex ;
e) fibres à bicomposants de polypropylène / polyéthylène d'une résistance de 3,3 à 17 dtex.

**3.** Nappe fibreuse pour la répartition d'un liquide selon la revendication 1 ou 2, **caractérisée en ce que** les fibres sont orientées majoritairement dans la direction longitudinale correspondant à la direction de fabrication et de traitement de la nappe (direction de la machine 1, 1', 1").

4. Nappe fibreuse pour la répartition d'un liquide selon une des revendications 1 à 3, **caractérisée en ce que** les fibres ont une longueur de 30 à 80 mm.

5. Nappe fibreuse pour la répartition d'un liquide selon l'une quelconque des revendications précédentes, **caractérisée en ce que** les fibres à bi-composants sont du type à âme / revêtement.

6. Nappe fibreuse pour la répartition d'un liquide selon l'une quelconque des revendications précédentes, **caractérisée en ce que** la surface de la nappe tournée en cours d'utilisation vers une couche d'absorption est lissée au moyen des cylindres de calandrage chaud ou d'un tambour de tamisage.

7. Nappe fibreuse pour la répartition d'un liquide selon l'une quelconque des revendications précédentes, **caractérisée en ce qu'**elle présente une épaisseur de 0,3 à 3 mm, de préférence de 1 à 2,5 mm.

8. Nappe fibreuse pour la répartition d'un liquide selon l'une quelconque des revendications précédentes, **caractérisée par** un motif d'impression (A, B, C ; figure 4) qui est obtenu au moyen de la calandre d'impression, qui présente une profondeur de gravure du cylindre à motifs d'au moins 1 mm et de préférence d'environ 1,2 mm.

9. Nappe fibreuse pour la répartition d'un liquide selon l'une quelconque des revendications précédentes, **caractérisée en ce que** le motif d'impression (A, B, C ; figure 4) comporte de 1 à 80 et de préférence de 2 à 15 impressions (4).

10. Nappe fibreuse pour la répartition d'un liquide selon l'une quelconque des revendications précédentes, **caractérisée en ce que** les impressions (4) sont formées de bâtonnets ou de losanges ou d'ellipses allongées dont la longueur correspond au moins à 1,5 fois la largeur.

11. Nappe fibreuse pour la répartition d'un liquide selon la revendication 10, **caractérisée en ce que** l'axe longitudinal (3) de l'impression (4) fait un angle compris entre $\pm$ 75° et de préférence entre $\pm$ 65° par rapport à l'axe de la machine (1, 1', 1") de fabrication de la nappe.

12. Nappe fibreuse pour la répartition d'un liquide selon la revendication 10, **caractérisée en ce que** l'axe longitudinal de l'impression (4) est situé à peu près dans la direction de la machine (1, 1', 1") fabriquant la nappe.

13. Nappe fibreuse pour la répartition d'un liquide selon l'une des revendications 1 à 9, **caractérisée en ce que** les impressions (4) sont constituées de rectangles qui sont disposés de manière telle qu'une diagonale est située à peu près dans la direction de la machine (1, 1', 1") fabriquant la nappe.

Prüfapparatur (Plexiglaskörper)

Fig 1

Fixpunkt

Folie

Gewichte    Gewichte

*a*

*c*

205m

Einfüllstutzen *a*

Windel *b*

**Fig 2**

630 mm

Draufsicht

*c*

28mm    32mm

*a*

**Fig 3**

Fig. 4

Fig. 5

Fig. 6